(19) Europäisches Patentamt
European Patent Office
Office européen des brevets

(11) **EP 3 988 125 A1**

(12) **EUROPEAN PATENT APPLICATION**

(43) Date of publication:
**27.04.2022 Bulletin 2022/17**

(21) Application number: **20207974.5**

(22) Date of filing: **17.11.2020**

(51) International Patent Classification (IPC):
**A61L 2/00** (2006.01)      **A61L 2/10** (2006.01)

(52) Cooperative Patent Classification (CPC):
**A61L 2/0047; A61L 2/10;** A61L 2202/14

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.10.2020 KR 20200137298**

(71) Applicant: **Aden Hygiene, Ent.**
**Cheongju-si, Chungcheongbuk-do 28160 (KR)**

(72) Inventors:
• **CHOI, Woo-Sung**
**16507 Gyeonggi-do (KR)**
• **CHO, Meoungwhan**
**16990 Gyeonggi-do (KR)**
• **LEE, Seogwoo**
**18385 Gyeonggi-do (KR)**

(74) Representative: **Schweitzer, Klaus**
**Plate Schweitzer Zounek**
**Patentanwälte**
**Rheingaustrasse 196**
**65203 Wiesbaden (DE)**

(54) **STERILIZATION APPARATUS USING UV LIGHT SOURCE HARMLESS TO HUMAN BODY**

(57)      The present invention provides a UV sterilization module capable of suppressing a generation of ozone inside and outside a device, and emitting UV rays having a wavelength of near 200 to 230 nm, in particular UV rays having a wavelength of 222 nm at a high luminous intensity, and a UV sterilization apparatus using the UV sterilization module. According to the UV sterilization apparatus of the present invention, bacteria or viruses existing on a surface of the biological tissue may be selectively UV sterilized without harming animal cells such as human cells, and a generation of ozone harmful to the skin may be reduced.

[FIG. 1]

EP 3 988 125 A1

## Description

[RELATED APPLICATIONS]

This application claims priority to Korean Patent Application No. 10-2020-0137298, filed on October 22, 2020 in the Korean Intellectual Property Office, the entire disclosure of which is incorporated herein by reference.

[BACKGROUND OF THE INVENTION]

1. Field of the Invention

The present invention relates to an ultraviolet (UV) sterilization module using a UV light source harmless to a human body and a UV sterilization apparatus including the same, and more specifically, to a UV sterilization apparatus using a UV light source harmless to a human body, which is harmless to animal cells, and is capable of proximately irradiating an object with sterilization light even in a space where there are humans, and thereby improving utilization.

2. Description of the Related Art

As a sterilization method, sterilization using heat/steam, sterilization by filtration, chemical sterilization, and sterilization by irradiation with UV rays are known in the art. UV sterilization using UV rays is performed by irradiating DNA in cells of a target organism such as bacteria and viruses with UV rays to sterilize the same. Specifically, DNA in the cell absorbs UV rays near 260 nm, which exhibit the highest absorption properties, destroys a genetic code of the DNA, and inactivates the target organism by preventing normal proliferation and metabolism of the cells from being performed. Conventionally, a technique for performing sterilization using a low-pressure mercury lamp exhibiting a strong emission spectrum at a wavelength of around 254 nm has been widely used in the UV sterilization field.

However, there is a disadvantage known in the art that, if the human body is directly irradiated with UV rays in such a wavelength range, various side effects may occur to the human body. If the human body is exposed to UV rays having a wavelength of 254 nm, they transmit through the stratum corneum of the epidermis, reach the granular layer or stratum spinosum, and in some cases, reach the basal layer, and are absorbed by the DNA of the cell existing in these layers to destroy or denature the DNA, thereby causing an increase in the possibility of skin cancer.

In a case of UV rays having a wavelength of 200 to 230 nm, an absorption rate of the skin and eyes is very low, and thereby they do not cause side effects even when exposed to UV rays. The reason is that, when irradiating the human body with UV rays in the above wavelength range, they are absorbed into the stratum corneum of the skin and are not transmitted to the basal layer side. Since keratinocytes contained in the stratum corneum are cells which do not have a cell nucleus, DNA does not exist therein. For this reason, even when irradiating with UV rays having a wavelength of about 250 nm, since they are absorbed by keratinocytes, there is almost no risk that DNA is destroyed.

Therefore, in a sterilization apparatus capable of directly performing UV sterilization on a living body such as skin, it is necessary to develop a device capable of selectively irradiating with UV rays in a wavelength range of 200 nm to 230 nm, in particular, a wavelength region of 222 nm known as Far-UVC, which have recently been found to be harmless to animal cells while being capable of obtaining UV sterilization effects for microorganisms to be sterilized, and a lot of research and development have been conducted on the basis of the finding.

However, in the case of the existing similar UV irradiation device, there is a problem that residual wavelengths (wavelengths other than a wavelength range of 200 to 230 nm) that is harmful to the human body may not be completely removed, thereby causing damage to the skin and cornea.

[Prior Art Document]

[Non-Patent Document]

(Non-Patent Document 1) Far-UVC light: A new tool to control the spread of airborne-mediated microbial diseases, David Welch, Manuela Buonanno, Veljko Grilj, Igor Shuryak, Connor Crickmore, Alan W. Bigelow, Gerhard Randers-Pehrson, Gary W. Johnson & David J. Brenner, Scientific Reports volume 8, Article number: 2752 (2018)

[SUMMARY OF THE INVENTION]

In consideration of the above-mentioned circumstances, it is an object of the present invention to provide a UV sterilization module capable of suppressing a generation of ozone inside and outside a device, and emitting UV rays having a wavelength of near 200 to 230 nm, in particular UV rays having a wavelength of 222 nm at a high luminous intensity.

In addition, another object of the present invention is to provide a UV sterilization apparatus using the UV sterilization module.

In order to achieve the above objects, according to an aspect of the present invention, there is provided an ultraviolet (UV) sterilization module including: a light source unit which includes a UV light source configured to emit a light including UV rays having a wavelength of 200 to 230 nm; a housing unit configured to house the UV light source; and a light guide unit which is configured to guide the light from the UV light source, and is blocked by an irradiation window allowing transmittance of UV rays, wherein the UV light source unit includes a distributed Bragg reflector (DBR) filter configured to selectively emit only peaks including a wavelength of 222 nm.

In one embodiment of the present invention, the UV light source may be one or more selected from the group consisting of an excimer lamp, an arc discharge lamp, or a UV-C LED.

In one embodiment of the present invention, the excimer lamp may include one or more excimers selected from the group consisting of ArF, KrBr, KrCl, KrF, and Xel.

In one embodiment of the present invention, the UV sterilization module is configured to directly irradiate skin with a radiation light thereof.

In addition, according to another aspect of the present invention, there is provided an ultraviolet (UV) sterilization apparatus including the UV sterilization module.

Further, according to another aspect of the present invention, there is provided an ultraviolet (UV) sterilization method including: performing, by the above UV sterilization apparatus, one or more of sterilization, deodorization, and removal of organic contaminants by directly irradiating skin of human or animal with UV rays emitted from the UV sterilization apparatus.

In one embodiment of the present invention, a light emission method of the excimer lamp may be a light emission method of dielectric barrier discharge.

In one embodiment of the present invention, it is possible to provide a UV sterilization module capable of irradiating with only a target wavelength at a high intensity by further including a filter technique for effectively removing residual wavelengths (wavelengths other than 222 nm) harmful to the human body.

In one embodiment of the present invention, the DBR filter may include an upper DBR and a lower DBR in a laminated structure, and a material of each layer of the upper DBR and the lower DBR is selected from the group consisting of Si3N4, SiO2, or ZnO.

In one embodiment of the present invention, each layer of the upper DBR and the lower DBR may have a thickness of 5 nm to 500 nm.

In one embodiment of the present invention, each of the upper DBR and the lower DBR may have a laminated structure of 5 to 100 layers.

In one embodiment of the present invention, one or more moth eye lenses are installed on one or more of LED modules of the UV-C LED light source.

In one embodiment of the present invention, the UV sterilization apparatus further includes a safety device, wherein the safety device includes a detector configured to measure one or more wavelength lights selected from the group consisting of UVC, UVB, UVA, and PUVA.

In one embodiment of the present invention, the UV sterilization apparatus may further include an ozone removal device, and the ozone removal device may include a UV radiation unit and a photocatalytic filter.

According to the UV sterilization apparatus of the present invention, bacteria or viruses existing on a surface of the biological tissue may be selectively UV sterilized without harming animal cells such as human cells, and a generation of ozone harmful to the skin may be reduced, as well as by simultaneously irradiating with visible light having a high light density, a site to be irradiated can be viewed, such that the UV irradiation range may be visually identified.

Another effect of the UV sterilization apparatus of the present invention is that both a space sterilization function that sterilizes and purifies microbial particles contained in the air existing in the indoor space and a surface sterilization function that sterilizes and disinfects the object surface in the indoor space may be performed simultaneously, and the object may be proximately irradiated with sterilization light even in a space where there are humans, and thereby improving utilization.

[BRIEF DESCRIPTION OF THE DRAWINGS]

The above and other objects, features and other advantages of the present invention will be more clearly understood from the following detailed description taken in conjunction with the accompanying drawings, in which:

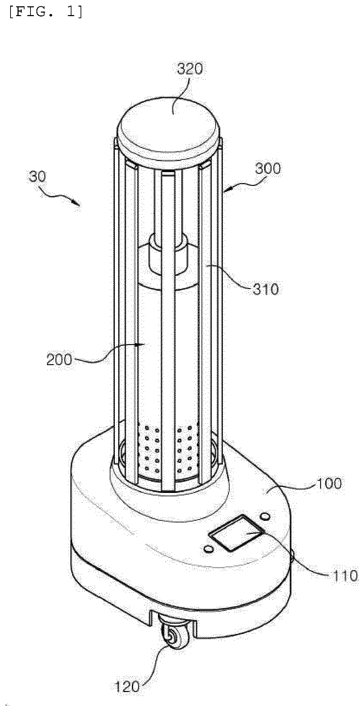

    FIG. 1 is a perspective view schematically illustrating an appearance of an indoor air sterilization and purification apparatus according to an embodiment of the present invention;

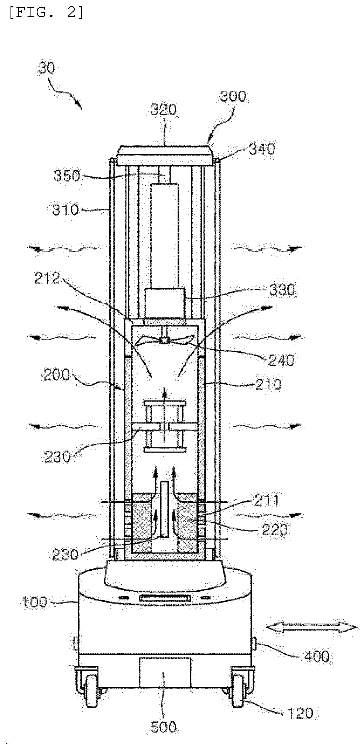

    FIG. 2 is a view conceptually illustrating a configuration of the indoor air sterilization and purification apparatus according to the embodiment of the present invention;

FIG. 3 is a view conceptually illustrating an inner structure of the indoor air sterilization and purification apparatus according to the embodiment of the present invention;

FIG. 4A is a reference view for a light emission method of the present invention, and FIG. 4B is a photograph taken a spark-shaped light emission form at the time of implementation of the present invention;

FIG. 5 is a schematic view of a DBR filter of the present invention;

FIG. 6 is a graph illustrating a reflected wavelength range of the DBR filter of the present invention;

FIG. 7 is a graph illustrating a sensitivity of Far-UVC sensing device for each wavelength of the present invention; and

FIG. 8 is a graph illustrating a reduction in acetaldehyde per hour by comparing the UV sterilization module of the present invention with a conventional UV-Lamp.

[DETAILED DESCRIPTION OF THE INVENTION]

[UV sterilization module]

A UV sterilization module of the present invention includes: a light source unit which includes a UV light source configured to emit a light including UV rays; a housing unit configured to house the UV light source; and an optical filter configured to adjust a wavelength of the emitted UV rays, wherein a light guide unit is blocked by an irradiation window allowing transmittance of UV rays.

Dimensions of the UV sterilization module may be optionally designed to suit the use and size of the sterilization apparatus, and the light source may have a length of 50 to 85% of a total module length.

Hereinafter, each component of the UV sterilization module will be described in detail.

[Light source unit]

In sterilization by UV rays including sterilization of skin, UV rays having a wavelength of 200 to 230 nm are used. The most preferred single wavelength is 222 nm. As a UV light source generating such a wavelength, an excimer lamp, an arc discharge lamp, or a UV-C LED may be used.

When the UV light source is an excimer lamp, any excimer lamp may be used so long as it is provided with a light emitting tube of a double pipe structure, which includes a cylindrical outer tube and a cylindrical inner tube having an outer diameter smaller than an inner diameter of the outer tube disposed along a tube axis in the outer tube, and is made of a material such as a synthetic quartz glass, fused quartz glass and the like.

In the light emitting tube, both ends of the outer tube and the inner tube are adhered by an adhesive member, respectively, to form a ring-shaped inner space between the outer tube and the inner tube, and a discharge gas is entrapped in the inner space.

In the light emitting tube, a mesh-shaped outer electrode is installed on an outer circumferential surface of the outer tube, and a membrane-shaped inner electrode is installed on an inner circumferential surface of the inner tube, and then the outer electrode and the inner electrode are connected to a high frequency power supply, respectively. In the excimer lamp, by applying a high frequency high voltage between the outer electrode and the inner electrode from a high frequency power source, excimer discharge occurs in the inner space and excimer light is emitted.

The excimer lamp may adjust a wavelength range of the emitted light according to types of the discharge gas, and the discharge gas to obtain a radiation light having a central wavelength around 200 nm may be argon fluoride (ArF, central wavelength 193 nm), krypton bromide (KrBr, 207 nm), krypton chloride (KrCl, 222 nm), krypton fluoride (KrF, 248 nm), xenon iodide (XeI, 253 nm), and chlorine ($Cl_2$, 259 nm). As the discharge gas, argon fluoride gas, krypton bromide gas, and krypton chloride gas are preferably used, and krypton chloride gas is most preferably used.

In addition, the excimer lamp may further include a reflective surface made of aluminum or formed by applying a UV light emitting phosphor having a peak wavelength in a wavelength range of 200 to 250 nm to the inner circumferential surface of the light emitting tube.

When the light source is a UV-C LED, the UV-C LED may adjust the wavelength range of the emitted light according to a configuration of the LED, such that radiation light (UV rays) having a central wavelength around 200 nm may be obtained. Most preferably, the LED is configured to emit a wavelength range of 200 to 230 nm. When the light source is a UV-C LED, the UV light source unit may further include a reflective surface.

When the light source is an arc discharge lamp, it may be configured as a light source unit including a short-circuit arc discharge lamp (hereinafter, briefly referred to as a "discharge lamp"), and a condensing optical system having a reflector disposed so as to surround the discharge lamp. The discharge lamp may be used to emit light having a continuous spectrum over a wavelength region of 200 nm to 500 nm.

The discharge lamp is a so-called both-end sealed type lamp, and includes, for example, a valve which has an oval-spherical light emitting tube portion forming a light emitting space, and a sealed tube portion extending outwardly in a tube axis direction in succession at both ends of the light emitting tube portion, and is made of glass (Suprasil F310). A pair of electrodes including a positive electrode and a negative electrode are disposed inside the light emitting tube portion to face each other. A distance between the positive electrode and the negative electrode may be 1 to 10 mm.

In the light emitting tube portion, mercury is sealed as a light emitting gas, and a sealing pressure (mercury vapor pressure) of the light emitting gas may be $1 \times 10^7$ Pa. The positive electrode may be made of tungsten. The negative electrode may be made of a tungsten sintered body impregnated with a reverse electron-emitting material (emitter material) such as valium oxide (BaO) or calcium oxide (CaO).

In the discharge lamp, when a voltage with a predetermined magnitude is applied between the positive electrode and the negative electrode, arc discharge is generated between the positive electrode and the negative electrode, and the discharge lamp is turned on.

The reflective surface is disposed so as to surround a periphery of the light emitting tube portion on the negative electrode side of the discharge lamp. It is preferable that the reflective surface has reflectivity for light having a wavelength of 230 nm or less and light having a wavelength of 400 nm or more, and is preferably composed of a metal vapor deposition film made of aluminum or a dielectric multilayer film to increase a reflectance of light having a wavelength of 230 nm or less.

Light emitted from the discharge lamp constituting the light source unit is reflected by the reflective surface of the reflector, is condensed to a second focal point through the optical filter, and is made incident on an incident end face of an incident optical system. The light made incident on the incident optical system is irradiated to a site to be irradiated through an optical fiber, and thereby performing sterilization and disinfection on organisms to be sterilized such as bacteria existing on the site to be irradiated.

In the sterilization apparatus of the present invention, a double cylindrical lamp may be adopted. In the conventional RF discharge method, it is possible to obtain uniform light emission by applying glow discharge, but there is a disadvantage in that an intensity of the desired peak wavelength is weak, but this is compensated for in the dielectric barrier discharge in the present invention. It is the same as the dielectric barrier discharge in an aspect that an outer wall is made of a dielectric material such as glass and the electrode is installed on an outside. However, by using a microplasma discharge method, light emission in a form of a thread may be obtained.

The UV sterilization module of the present invention ensures a sterilization ability for a distant space. By applying an optical lens to the light source emission unit, light is condensed to enable sterilization of the distant space. As the optical lens, a moth eye lens may be used, and one or more moth eye lenses may be installed on each LED, and preferably, one moth eye lens is installed. When simultaneously irradiating with UV rays from LED light sources adjacent to each other, a mutual condensing action of the moth eye lens occurs. When the optical lens is not installed, an effective distance (Wd) is about 30 mm, but when the optical lens is applied, the effective distance is increased more than 10 times, and thereby maximizing a disinfection ability at a distance. The sterilization module of the present invention may be applied to a Far UV-C sterilization apparatus that maximizes the optical power of a desired region.

[Optical filter]

The UV sterilization module of the present invention may further include an optical filter in the light source unit or the light guide unit.

In order to achieve a UV sterilization module which irradiates skin with light having a spectral line width, the light source unit needs an optical filter which absorbs, reflects, and attenuates light having different wavelengths so as to irradiate light having a desired wavelength range.

The optical filter may also be a band pass filter (BPF) that reduces light having a wavelength of 230 to 300 nm, a DBR filter, or a UV absorption member.

In the radiation light of the light source unit, light of a shorter wavelength and a longer wavelength around the central wavelength is generated in small amounts. When UV rays having a wavelength of less than 190 nm are included in the radiation light, the UV rays having the wavelength are absorbed by oxygen, and ozone (O3) may be generated. Since the ozone generated in a high concentration negatively affects the human body, it is necessary to suppress the generation of ozone when directly applying it to a living body for the purpose of skin sterilization treatment, etc. Accordingly, the light source unit may additionally include the ozone filter for absorbing UV wavelengths of less than 190 nm or preventing the generated ozone from being discharged.

As a means for preventing ozone generation of the present invention, an ozone decomposition device of photocatalysis method may also be used. Preferably, a TiO2 photocatalysis method may be used. Air is sucked into an inlet and then passes through a TiO2 filter irradiated with a wavelength of 365 nm, followed by passing through a high efficiency particulate air (HEPA) filter. The air passing through the HEPA filter is irradiated with UV rays having a wavelength of 275 nm. In the TiO2 filter irradiated with a wavelength of 365 nm, the generated ozone is decomposed into oxygen again. Herein, the HEPA filter has functions of sterilization, deodorization, PM2.5 particle removal, and dust removal, while UV rays having a wavelength of 275 nm have an effect of sterilizing microorganisms such as bacteria or viruses.

The light wavelength of the UV sterilization module of the present invention may be 200 nm to 230 nm, and preferably 210 to 225 nm. From the viewpoint of suppressing the effect on DNA upon UV irradiation, it is preferable that the wavelength of UV rays is short. However, since UV rays having a wavelength of less than 200 nm are absorbed by oxygen in the air, an amount of light reaching the skin is greatly reduced, such that the sterilization effect cannot be guaranteed. Since a light wavelength of more than 230 nm may cause side effects such as skin damage, it is preferable that the light source is equipped with a filter which blocks light having a wavelength longer than 230 nm.

However, when using a filter which blocks all light having a wavelength longer than 230 nm, only UV rays in the invisible region are irradiated on the surface of biological tissues. Therefore, in order to solve a problem that a position with which UV rays are irradiated cannot be visually determined, the present invention provides a UV sterilization apparatus including the optical filter which transmits light in a wavelength region of from 200 nm to 230 nm (hereinafter, "A wavelength light"), blocks light in a wavelength region of more than 230 nm to 400 nm (hereinafter, "B wavelength light"), and attenuates light in a wavelength region of more than 400 nm to 500 nm (hereinafter, "C wavelength light"), thus to emit A wavelength light and C wavelength light, etc.

The optical filter of the present invention may include: a first attenuation filter which transmits light having a wavelength of 200 to 230 nm, blocks light having a wavelength of 230 to 400 nm, and attenuates light having a wavelength of 400 nm or more, and for example, absorbs light having a wavelength of 230 to 400 nm; and a second attenuation filter which absorbs light having a wavelength of 400 nm or more. The optical filter may be configured in such a way that the first attenuation filter and the second attenuation filter are installed by laminating on the same glass substrate. The first attenuation filter may be a band pass filter having a central wavelength of 214 nm, and an optical density (OD) value of 40 or more for light having a wavelength of 230 to 400 nm. The second attenuation filter may be a short pass filter having a cutoff on/off wavelength of 400 nm, and an OD value of 20 or more for light having a wavelength of 400 nm or more, and a block wavelength range of 400 nm to 500 nm.

In the filter, a ratio of light in a wavelength region of more than 400 nm to 500 nm to light in a wavelength region of 200 nm to 230 nm in the transmitted light that has transmitted through the filter may be 1:0.1 to 0.5.

The ratio of light having a wavelength of 400 nm or more to light having a wavelength of 200 to 230 nm is expressed as an area ratio integrated with respect to the wavelength in the spectrum of the transmitted light that has transmitted through the optical filter. When the ratio of light having a wavelength of 400 nm or more to light having a wavelength of 200 to 230 nm exceeds 1:0.5, the biological tissues are strongly irradiated with blue light, and high safety for animal cells may not be secured. In addition, when the ratio of light having a wavelength of 400 nm or more to light having a wavelength of 200 to 230 nm is less than 1:0.1, the site to be irradiated may not be clearly viewed, and it may be difficult to visually identify the UV irradiation range.

In the filter, a ratio of light in a wavelength region of 701 nm to 800 nm to light in a wavelength region of 200 nm to 230 nm in the transmitted light that has transmitted through the filter may be 1:0.1 or less. When the ratio of light having a wavelength of 701 to 800 nm to light having a wavelength of 200 to 230 nm exceeds 1:0.1, it may be difficult to secure high safety for animal cells by irradiating the biological tissue with near-infrared light.

The band pass filter may reduce an intensity ratio of light to 10% or less, and light having a wavelength of 200 to 230 nm transmitted through the band pass filter may be reflected by the UV absorption member to absorb UV rays.

Specifically, the intensity ratio of light after transmitting through the band pass filter is measured as in Equation 1 below.

[Equation 1]

Light intensity ratio (%) = {Intensity of UV rays in a wavelength region of 230 to 300 nm / Intensity of UV rays in a wavelength region of 200 to 230 nm} × 100

According to the principle of the band pass filter, when exceeding a certain incidence angle, light blocking efficiency is decreased. When the incidence angle of light is 0° (made incident perpendicular to an incidence surface), light in a wavelength region near 220 nm is transmitted, and UV rays of 230 to 300 nm are blocked. Meanwhile, when the incidence angle of light is 40°, light in a wavelength region of about 210 nm or more is blocked, and light in a wavelength region of 280 nm or more is transmitted. In addition, when the incidence angle of light is 60°, light in a wavelength region of about 200 nm or more is blocked, and light in a wavelength region of 260 nm or more is transmitted.

In light made incident on the band pass filter at a low angle of 0° to 40°, B wavelength light is selectively absorbed and blocked by the band pass filter, and A wavelength light is not blocked but transmits through the band pass filter. Light including A wavelength light that has transmitted through the band pass filter is directly emitted from an irradiation window or made incident on a light guide unit at a high angle, and is reflected to an inner surface of the UV absorption member constituting the light guide unit and is emitted from the irradiation window.

Meanwhile, among the radiation light of the excimer lamp made incident on the band pass filter, B wavelength light from the high-angle component light made incident on the band pass filter at a high angle exceeding 40° is included in the light transmitting through the band pass filter. Light including the transmitted B wavelength light is made incident on the light guide unit at a low angle, and thereby the light is absorbed and blocked by the UV absorption member of the light guide unit, or transmitted through the UV absorption member and is emitted toward the outside of the light guide unit. In addition, A wavelength light included in the high-angle component light is absorbed and blocked by the band pass filter, or even if it transmits through the band pass filter without being blocked, the light is absorbed and blocked by the UV absorption member together with the B wavelength light included in the light, or transmits through the UV absorption member and is emitted toward the outside of the light guide unit. Accordingly, the B wavelength light is blocked or absorbed into the low-angle component light and the high-angle component light, thereby it is possible to suppress the B wavelength light from being emitted from the irradiation window.

As the UV absorption member, glass such as quartz glass, soda lime glass or fluorosilicic acid glass may be used. Since the glass exhibits Fresnel reflection properties, light made incident on the glass at a high angle is reflected, and light made incident on the glass at a low angle is absorbed or transmitted. Herein, the high-angle component light associated with the bandpass filter is made incident on the UV absorption member at a low angle, and the high-angle component light is absorbed or transmitted by the UV absorption member. Meanwhile, the low-angle component light is not absorbed or transmitted by the UV absorption member, but reflected toward the irradiation window by the UV absorption member, such that harmful UV rays leaking out of the device may be reliably suppressed. In addition, a user may observe the visible light of the excimer lamp from the outside, and high convenience may be obtained.

In the UV sterilization apparatus of the present invention, the band pass filter is installed between the light guide unit and the lamp housing unit or at a tip portion on a light emitting side of the light guide unit, and an inner surface of the light guide unit may be made of a UV absorption member which absorbs wavelengths of light harmful to the human body. A part of the B wavelength light included in the light from the UV light source is selectively blocked by the band pass filter, and the remaining B wavelength light is absorbed or transmitted by the UV absorption member. As a result, in the light emitted from the UV sterilization apparatus of the present invention, it is possible to decrease the luminous intensity of the B wavelength light without significantly reducing the luminous intensity of the UV rays including the A wavelength light.

The optical filter may be a DBR filter, and preferably a DBR filter including a ZnO layer. The DBR filter may be a filter having a layered structure consisting of an upper DBR, a space layer, a lower DBR, and a substrate, and each layer of the upper DBR may have a multi-layered structure with a uniform height, and each layer of the lower DBR may also have a multi-layered structure with a uniform height. Each layer of the upper or lower DBR may have a thickness of 5 nm to 500 nm, and preferably 10 nm to 100 nm. The upper or lower DBR may have a layered structure of 2 to 300 layers, preferably 5 to 100 layers. The material of each layer of the upper or lower DBR may be one or more selected from the group consisting of ZnO, $SiO_2$, and $Si_3N_4$. Each layer may have a structure in which several materials are alternately laminated one by one, but two or more continuous layers may be selected as the same material. The material of the substrate may be stable minerals such as sapphire or crystal.

[Lamp housing unit]

The light source and the reflective member of the present invention are supported and fixed by base members disposed at one end or both ends of the light source and the reflective member. Specifically, each of the base members may be formed in a substantially circular cylindrical body. A cross section of the circular cylindrical body perpendicular to a cylinder axis thereof may be a ring or cylindrical shape having an inner diameter slightly smaller than the inner diameter of the inner tube of the light source and slightly larger than the outer diameter of the reflective member. When the base member is a ring or a cylindrical shape, cooling efficiency of the light source may be improved.

A ring or cylindrical engaging part having an inner diameter slightly smaller than the inner diameter of the inner tube of the light source may be installed on an inner surface of the base member so as to protrude with being communicated with the inside the ring of the base member. Further, the ring or cylindrical engaging part is installed on an outer surface of the base member so as to protrude with being communicated with the inside the ring of the base member. In the base member, the inwardly protruding engaging part is inserted into the inner tube of the light source, such that the light source is fixed to the base member.

In the lamp housing unit, the UV light source is disposed so as to extend on the same axis as the cylinder axis of the peripheral wall part. Specifically, one or more UV light source support parts having an outer circumferential shape as an inner circumferential shape in a cross section perpendicular to the peripheral wall part in an axial direction are installed spaced apart from each other inside the peripheral wall part of the lamp housing unit. Therefore, the engaging parts of the base member of the UV light source are respectively engaged to engaging holes formed in the support part of the UV light source unit, such that the UV light source is supported and fixed in the lamp housing unit. The peripheral wall part is made of a material having a glossiness, for example, black alumite-treated aluminum. Further, it is preferable that a reflective layer is installed in a region on the inner surface of the peripheral wall part facing the light emitting portion of the excimer lamp.

The lamp housing unit in which the UV light source is housed may further include a peripheral wall part consisting of a square column, a cylinder, and a polygonal column, and a supply fan and an exhaust fan for circulating cooling air for cooling the light sources in the lamp housing unit installed at both ends of the peripheral wall part. Herein, it is preferable that an ozone filter is installed in one exhaust path of the exhaust fan and the supply fan.

[Light guide unit]

The light guide unit for guiding light from the excimer lamp may have a polygonal or spherical shape including a cylinder, a square column, a hexagonal column, and the like, and may have a three-dimensional shape modified from a basic three-dimensional figure.

The light guide unit for guiding light from the UV light source has a rectangular cylindrical portion, and a light guide window allowing transmittance of UV rays having an outer circumference shape similar to an inner circumference shape of the base end portion is fitted and fixed to the base end portion of the cylindrical portion. Meanwhile, the tip portion of the cylindrical portion is blocked by the irradiation window allowing transmittance of UV rays. Specifically, the irradiation window having an outer circumferential shape similar to the inner circumferential shape of the tip portion is fitted and fixed to the tip portion of the cylindrical portion.

The length of the light guide unit may be a size in which all of the high-angle component light associated with the band pass filter is directly made incident on the inner surface of the light guide unit among the light emitted from the band pass filter. When the length of the light guide unit is too short, a part of the high-angle component light associated with the band pass filter among the light emitted from the band pass filter is not made incident on the inner surface of the light guide unit, but directly made incident on the irradiation window and is emitted from the irradiation window. Meanwhile, when the length of the light guide unit is too long, an amount of light made incident on the UV absorption member is increased compared to the low-angle component light associated with the band pass filter of A wavelength light, such that loss of light due to light absorption in the UV absorption member is increased, and thereby the light reaching the irradiation window may be decreased.

A color of the light guide unit may be entirely transparent, may be partially transparent, may be entirely translucent or partially translucent, may include a portion reflecting light of an excimer lamp, and may be a mixture thereof.

The light guide unit may be a light guide window allowing transmittance of UV rays or may separately have a light guide window, or the entire light guide unit may be a light guide window or an irradiation window. The lamp housing unit and the light guide unit may be integrally provided as a whole, and the lamp housing unit and the light guide unit may be separately provided.

A portion that reflects light from the excimer lamp may be a reflective layer made of a high-brightness aluminum plate or an aluminum vapor deposition film.

An oxygen-containing layer formation space, in which the reflective layer installed on the inner circumferential surface of the cylindrical portion in the light guide unit and the light guide window are divided by the irradiation window, is filled with a gas that absorbs UV rays to generate ozone, such that the oxygen-containing layer may be formed. The oxygen-containing internal gas may be air in the atmosphere or the like.

In the UV radiation apparatus of the present invention, the oxygen-containing layer is formed in the light guide unit which is blocked by the irradiation window allowing transmittance of UV rays, such that light from the UV light source which emits light including UV rays (light having a wavelength of 190 nm or less) of a wavelength absorbed by oxygen to generate ozone is emitted through the oxygen-containing layer. Therefore, light having a wavelength of 190 nm or less contained in the light from the UV light source is selectively absorbed into the oxygen-containing layer.

The oxygen-containing layer formation space may be an airtight blocked space in which ozone generated in the oxygen-containing layer does not leak at all to the outside. In addition, if an amount of ozone leaking to the outside, which can be generated in the oxygen-containing layer, is not large, high airtightness may not be required.

A thickness of the oxygen-containing layer, that is, a distance of the light guide window spaced apart from the irradiation window, may be determined depending on the configuration of the excimer lamp and the lighting conditions of the excimer lamp, wherein the thickness of the oxygen-containing layer is preferably 10 to 100 mm, and more preferably 30 to 60 mm. When the thickness of the oxygen-containing layer is less than 10 mm, the absorption ability of the oxygen-containing layer to a wavelength of 190 nm or less is decreased, such that a generation of ozone outside the apparatus cannot be sufficiently suppressed. Meanwhile, if the thickness of the oxygen-containing layer exceeds 100 mm, a heat retention effect by the oxygen-containing layer is increased, such that the excimer lamp is excessively heated due to the heat generated by lighting the excimer lamp and the service life of the excimer lamp may be shortened.

In addition, a separate ozone decomposition means may be disposed in the oxygen-containing layer formation space in the light guide unit. The ozone decomposing means may be a device such as a heater for thermally decomposing ozone generated in the oxygen-containing layer.

The irradiation window may be made of a material such as quartz glass. It is preferable that the irradiation window has a filter function that blocks light having a wavelength of 315 to 400 nm (hereinafter referred to as "harmful UV rays A") harmful to the skin. Specifically, it is possible to impart a filter function to the irradiation window by further including a filter layer that blocks harmful UV rays A and transmits light having a wavelength other than the above range on an inner surface or an outer surface of the irradiation window.

The light guide window may be made of a material such as quartz glass. Unlike the wavelength region (315 nm to 400 nm) of UV rays (harmful UV rays A) blocked by the irradiation window, it is preferable that the light guide window has a filter function to block light having a wavelength of 280 to 315 nm that is harmful to the skin (hereinafter, referred to as "harmful UV rays B"). Specifically, the inner surface or the outer surface of the light guide window may further include a filter layer that blocks harmful UV rays B and transmits light having a wavelength other than the above range, thereby imparting a filter function.

In the UV sterilization module of the present invention, for an irradiation amount (irradiation density) of light emitted from the discharge lamp to irradiate the biological tissue through the optical filter, it is preferable that an exposure amount calculated by the irradiation density and a irradiation time is a level that can sterilize a living body to be sterilized on a surface of the biological tissue. The irradiation amount of light varies depending on the irradiation time, but may be considered to be 5 to 420 mJ/cm$^2$, for example. The irradiation amount of light per hour may be 5 to 400 mW/cm$^2$, preferably 10 to 200 mW/cm$^2$, more preferably 20 to 100 mW/cm$^2$, and most preferably 30 to 50 mW/cm$^2$. When the irradiation amount of light is excessively large, light having a wavelength of 230 to 400 nm emitted from the light source and made incident on the optical filter is increased, such that light having a wavelength of 230 to 400 nm that is harmful to the living body may not be reliably blocked. Meanwhile, when the irradiation amount of light is excessively small, a long time is required for sufficient sterilization and disinfection of the organism to be sterilized.

[Ultraviolet sterilization apparatus]

The present invention provides a sterilization apparatus capable of reliably identifying a UV irradiation range by simultaneously irradiating visible light having a high optical density in the UV irradiation range so that the site to be irradiated may be clearly viewed.

The UV sterilization apparatus of the present invention may further include a disinfectant spray module capable of spraying an external preparation for skin, cosmetics, or disinfectant in addition to the UV sterilization module. The disinfectant may be one or more selected from the group consisting of povidone-iodine, benzalkonium chloride, hydrogen peroxide, glutaraldehyde, boric acid water, and nitrofurazone.

The UV sterilization apparatus of the present invention may include a sensor capable of detecting that a region to be irradiated with UV rays is inserted into the sterilization apparatus, and the light source may automatically emit the UV rays only for a predetermined time.

The present invention may provide a UV sterilization apparatus having an autonomous traveling function. Specifically, the present invention provides an indoor air sterilization and purification apparatus including: a driving unit which includes a plurality of wheels provided at a lower portion thereof to automatically travel in an indoor space; a circulation purification module mounted on the driving unit and configured to suck in an indoor air to filter, sterilize, or purify dusts or microbial particles contained in the indoor air and discharge the same; a sterilization and disinfection module which is mounted on the driving unit, includes a plurality of main germicidal lamps that emit sterilization light, wherein the main germicidal lamps are formed in a manner of adjusting arrangement positions thereof so that the main germicidal lamps are located close to an object to be disinfected, and is configured to irradiate a sterilization region between the object and the main germicidal lamps with UV-C; distance detection sensors mounted on the driving unit and the sterilization and disinfection module to detect a distance to an object existing in the indoor space therefrom; and a controller configured to control operations of the driving unit and the sterilization and disinfection module based on detection results of the distance detection sensors, wherein the main germicidal lamp is a UV-C LED or a UV-C lamp having a wavelength of 200 to 230 nm.

The circulation purification module may further include a disinfectant spray module provided at a rear end of the air outlet.

The sterilization and disinfection module may further include a disinfectant spray module provided at an upper portion of the sterilization base body, or may further include a disinfectant spray module at one end of the main germicidal lamp.

The indoor air sterilization and purification apparatus of the present invention may further include a safety device at the time of UV irradiation. The safety device checks a status of Far UV-C by a detector or an optical sensor, displays the safety status of the apparatus by a display lamp such as LEDs, and imparts an interlock function on the apparatus so that UV rays of the apparatus are not irradiated in a state in which a danger may occur. To check the status of the Far UV-C, a high power application sensor package and a low power application sensor package may be applied using a UV photodiode, respectively, or a phosphor and a visible light diode may be simultaneously applied, thereby providing a safety device for the indoor sterilization and purification apparatus. As a sensor package, a high-power sensor package C3535, TOPKG, or the like produced by Seoul Viosys may be used, and a low-power sensor package SMD3528, SMD3020, SMD2016, or the like may be used.

Hereinafter, the present invention will be described in detail based on embodiments thereof, but the present invention is not limited to these embodiments.

According to one embodiment of the above UV sterilization module, in this UV radiation apparatus, by applying a high frequency high voltage to a pair of electrodes of the excimer lamp from the high frequency power source, excimer discharge occurs in the internal space, and excimer light according to the type of discharge gas is emitted from the outer circumferential surface of the outer tube as radiation light. The radiation light from the excimer lamp contains UV rays having a wavelength which is absorbed by oxygen to generate ozone. In addition, the radiation light of the excimer lamp is emitted from a UV ray emitting region on the outer circumferential surface of the outer tube, after a part thereof is reflected directly, and the other part is reflected by the reflective member. A part of the light emitted from the UV light emission region of the outer tube of the excimer lamp is reflected directly, and the other part is reflected to the inner surface of the peripheral wall part of the lamp housing unit, and then made incident on the light guide window of the light guide unit. Among the radiation light of the excimer lamp made incident on the light guide window, harmful UV rays B (UV rays having a wavelength region of 280 to 320 nm) are selectively blocked by the filter layer of the light guide window, such that light in wavelength regions other than the above range is transmitted. In addition, the light transmitted through the light guide window is made incident on the oxygen-containing layer blocked by the light guide window and the irradiation window, and light having a wavelength of 190 nm or less contained in the light is selectively absorbed into the oxygen-containing layer to generate ozone, and light from which 190 nm or less wavelength light has been removed is made incident on the irradiation window. Herein, when the oxygen-containing layer formation space in which the oxygen-containing layer is formed is a blocked space, light having a wavelength of 190 nm or less is absorbed into oxygen in the atmosphere, thus to generate ozone, but leakage of ozone to the outside of the apparatus is suppressed.

In addition, among the light made incident on the irradiation window, harmful UV rays A (UV rays in a wavelength region of 230 to 280 nm) are selectively blocked by the filter layer of the irradiation window, such that light in wavelength regions other than the above range is transmitted. As described above, from the irradiation window of the UV radiation apparatus, light having a wavelength of 190 nm or less and light, in which the luminous intensities of the harmful UV rays A and the harmful UV rays B are decreased, are emitted. In the lamp housing unit, when the supply fan and the exhaust fan are operated, the air outside the apparatus flows as cooling wind from one end where the supply fan is disposed to the other end where the exhaust fan is disposed.

In addition, the light emitted from the UV ray emission region of the outer tube of the excimer lamp is also absorbed into the oxygen-containing layer in the lamp housing unit to generate ozone. This ozone is heated by heat, etc. from the excimer lamp and rapidly decomposed (pyrolyzed) to generate oxygen or is adsorbed and removed by an ozone filter installed between the excimer lamp and the exhaust fan. Specifically, air is sucked into the inlet and then passes through a TiO2 filter irradiated with a wavelength of 365 nm, followed by passing through the HEPA filter. The air passing through the HEPA filter is irradiated with UV rays having a wavelength of 275 nm. In the TiO2 filter irradiated with a wavelength of 365 nm, the generated ozone is decomposed into oxygen again, and ozone discharged to the outside of the apparatus may be reduced.

Therefore, according to the UV radiation apparatus, it is possible to suppress a generation of ozone in the atmosphere outside the apparatus, and by selecting and using a discharge gas in the excimer lamp, UV rays in the wavelength range of 200 nm are emitted at a high luminous intensity. Furthermore, even when the UV radiation apparatus generates ozone inside the apparatus, it is possible to suppress the ozone from leaking out of the apparatus.

In one embodiment of the present invention, the above-described UV sterilization apparatus includes: a light source capable of emitting UV rays having a wavelength of 200 nm to 230 nm, and an insertion port for inserting a region to be irradiated into the apparatus. Further, if necessary, the inventive apparatus may be provided with a pedestal. The pedestal may be installed to adjust a distance between the light source and the object to be irradiated. In addition, since the UV irradiation apparatus is equipped with a sensor, such that power is automatically supplied to the light source by sensing when the object to be irradiated is inserted, and thereby the UV rays may be irradiated to the object only for a set time.

An indoor air sterilization and purification apparatus according to an embodiment of the present invention is an apparatus capable of simultaneously performing a space sterilization function, which is an air purification function for an indoor space, and a surface sterilization function, which is a sterilization and disinfection function for surfaces of objects in the indoor space, and includes a driving unit 100 (hereinafter, also referred to as an automatic traveling mobile body 100), a circulation purification module 200, a sterilization and disinfection module 300, a distance detection sensor 400, and a controller 500.

The driving unit 100 is a mobile body capable of automatically traveling in an indoor space, and is configured to detect an obstacle by itself and automatically travel without a driving operation by a user. The driving unit may include wheels 120 for movement mounted at a lower portion thereof, buttons for operation and manipulation by the user, and an operation display unit 110 for displaying images, which are mounted on one side thereof.

In addition, the driving unit 100 may be equipped with various parts for automatic traveling, such as a driving unit (not illustrated) to rotate the wheels 120, and in particular, a plurality of distance detection sensors (400) to detect a distance to an object 20 existing in the indoor space therefrom. The controller 500 may calculate a moving route of the automatic traveling mobile body 100 based on detection results of the distance detection sensors 400, and control the driving unit 100 to move without colliding with an obstacle such as the object 20 in the indoor space 10.

The circulation purification module 200 is mounted on the driving unit 100 to move along with the automatic traveling mobile body 100, and is configured to sterilize and purify fine particles and microbial particles contained in an indoor air by sucking in the indoor air.

The sterilization and disinfection module 300 is mounted on the driving unit 100 to move along with the automatic traveling mobile body 100, and includes a plurality of main germicidal lamps 310 that emit sterilization light. The main germicidal lamps 310 may be the inventive UV sterilization module, and are formed in a manner that arrangement positions of the main germicidal lamps 310 may be adjusted so that the main germicidal lamps 310 are located close to the object 20 to be disinfected. The distance detection sensor 400 may also be mounted on the sterilization and disinfection module 300, and the controller 500 may adjust a proximal moving distance of the main germicidal lamps 310 to the object 20 to be disinfected based on the detection results of the distance detection sensors 400.

Meanwhile, the controller 500 may control operations of the modules so as to be operated simultaneously in both or selectively in any one of a space sterilization mode that operates the circulation purification module 200 to purify the air in the indoor space 10, and a surface sterilization mode that operates the sterilization and disinfection module 300 to perform sterilization and disinfection on the surface of the object 20.

According to the above-described configuration, the indoor air sterilization and purification apparatus 30 according to the embodiment of the present invention may simultaneously perform the space sterilization mode through the circulation purification module 200 and the surface sterilization mode through the sterilization and disinfection module 300 to simultaneously perform sterilization and disinfection on the object in the indoor space 10.

More specifically, the controller may continuously operate the circulation purification module 200 so as to continuously perform the space sterilization mode in the indoor space, and operate the main germicidal lamp 310 so as to perform the surface sterilization mode in which the surface of the object 20 existing in the indoor space 10 is irradiated with the sterilization light while moving an automatic traveling mobile body with being operated in the space sterilization mode. At this time, in the surface sterilization mode, by moving the main germicidal lamp 310 close to the surface of the object 20 to be disinfected, the surface of the object 20 to be disinfected is proximately irradiated with the sterilization light, thereby effects of sterilizing and disinfecting the object surface may be improved.

For example, when a plurality of objects 20 exist in the indoor space 10, the indoor air sterilization and purification apparatus 30 according to the embodiment of the present invention detects the object 20 using the distance detection sensor 400 etc., and the controller 500 calculates the moving route of the driving unit 100 based on the detection results. Therefore, it is possible to move the entire region of the indoor space 10 so as to be located close to the object 20 along the moving route.

In this way, the indoor air sterilization and purification apparatus 30 moves the entire region of the indoor space 10, such that all objects 20 of the indoor space 10 are proximately irradiated with the sterilization light from the main germicidal lamp 310, and thereby the sterilization and disinfection function on the surface of the object 20 may be perfectly performed in full.

Meanwhile, the circulation purification module 200 is disposed on an upper portion of the driving unit 100, and the sterilization and disinfection module 300 may be disposed movably up and down in a form surrounding an upper portion and a side space of the circulation purification module 200.

The circulation purification module 200 may include: an air purification case 210 mounted on an upper portion of the automatic traveling mobile body 100 and having air inlets 211 and an air outlet 212 formed on one side and the other side thereof; an air filter 220 mounted inside the air purification case 210 to filter the air sucked through the air inlets 211; UV sterilization modules 230 configured to emit sterilization light into an inner space of the air purification case 210 to sterilize microbial particles contained in the air sucked through the air inlets 211; and a blowing fan 240 operated to form an air flow so that air is sucked and discharged through the air inlets 211 and the air outlet 212. Herein, the blowing fan 240 and the UV sterilization module 230 are operated by a control of the controller 500.

The air inlets 211 are formed in sides of a lower end portion of the air purification case 210, and the air outlet 212 is formed in an upper end portion of the air purification case 210, such that external air may be introduced into the inner space through the sides of the lower end portion of the air purification case 210 to be filtered, and then discharged to an outside through the upper end portion.

In this case, the air filter 220 may be applied as a filter capable of filtering both fine dusts and microbial particles, and may be formed in a cylindrical shape to allow air to pass therethrough from the sides to a center direction.

A plurality of UV sterilization modules 230 may be provided so as to sterilize both microbial particles filtered by the air filter 220 and microbial particles which are not filtered by the air filter 220 inside the air purification case 210. Herein, at least one lamp may be disposed to emit sterilization light toward the air filter 220 in a form of being inserted into a central portion of the air filter 220. The remaining lamps of the plurality of UV sterilization modules 230 may be disposed above the air filter 220 to sterilize the microbial particles passing through the air filter 220.

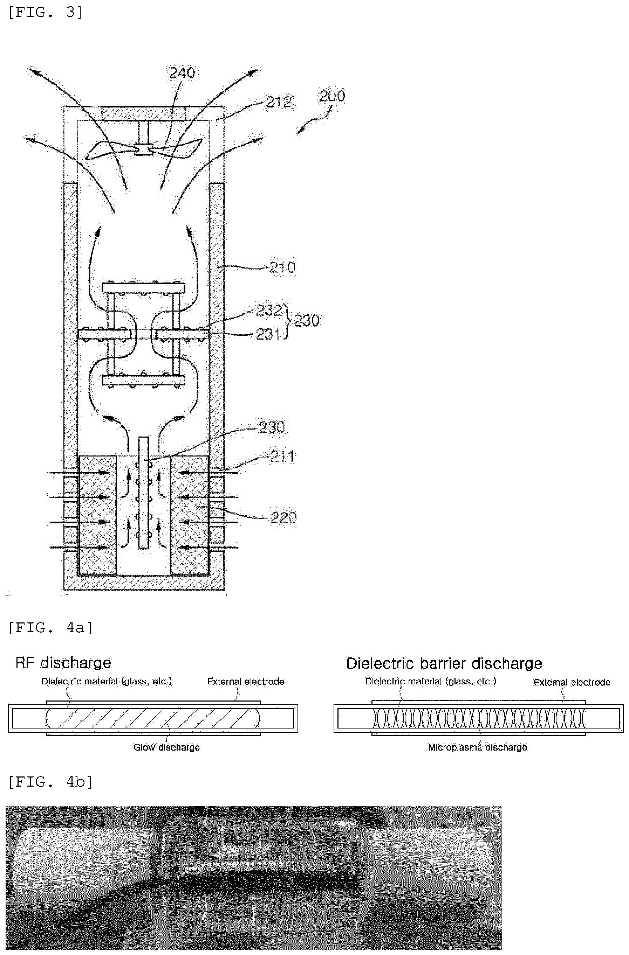

As shown in FIG. 3, these UV sterilization modules 230 may be formed in a form in which a plurality of UV LEDs 232 capable of emitting UV rays are mounted on a plate-shaped lamp substrate 231, and the plurality of UV sterilization modules 230 disposed above the air filter 220 may be formed in such a way that they are disposed above the air filter 220 to be vertically spaced apart from each other, and may be formed to guide the air flow passing through the air filter 220 in a zigzag direction.

To this end, the plurality of UV sterilization modules 230 may be disposed to be shifted from each other in a vertical direction inside the air purification case 210.

According to this structure, the microbial particles filtered by the air filter 220 are sterilized by the UV sterilization module 230 inserted in the central portion of the air filter 220, and the microbial particles passing through the air filter 220 flow in an upward zigzag direction by the plurality of UV sterilization modules 230 as shown in FIG. 4, such that the time at which the microbial particles stay inside the air purification case 210 is increased. Therefore, an exposure time at which the microbial particles are irradiated with the sterilization light emitted from the UV sterilization module 230 is increased, and thereby the sterilization function is improved.

The sterilization and disinfection module 300 according to an embodiment of the present invention includes: a sterilization base body 320 disposed above the air purification case 210; a main driving unit 330 configured to move the sterilization base body 320 up and down; and a plurality of main germicidal lamps 310 which are arranged along an outer circumference of the sterilization base body 320, move up and down along with the sterilization base body 320, extend longitudinally in a vertical direction, and have light emitting surfaces formed on outer surfaces thereof to emit sterilization light to an outer direction. Herein, the main driving unit 330 and the main germicidal lamp 310 are operated by a control of the controller 500.

Upper ends of the plurality of main germicidal lamps 310 are pivotably coupled to the sterilization base body 320 about a horizontal rotation shaft 340, and pivot driving units 360 are mounted on the sterilization base body 320, which independently pivot and move the plurality of main germicidal lamps 310 about the horizontal rotation shaft 340 to be operated by a control of the controller 500.

In addition, the main driving unit 330 may be configured to move the sterilization base body 320 up and down, as well as to rotate and move the sterilization base body 320 about the vertical rotation shaft 350 provided at the central portion thereof.

In this case, the main germicidal lamp 310 may have light emitting surfaces formed on an outer surface and an inner surface thereof, respectively, so as to emit the sterilization light in the outer direction and an inner center direction, respectively, with being arranged longitudinally in the vertical direction. For example, it may be formed in a form in which a lamp substrate 311 formed longitudinally in the vertical direction is disposed at the central portion, a plurality of UV LEDs 312 capable of emitting UV rays are mounted on both sides of the lamp substrate 311, respectively, and transparent protective covers 313 are mounted at the outside.

According to the above-described configuration, a plurality of main germicidal lamps 310 are arranged to be spaced apart from each other in a circumferential direction along the outer circumferential surface of the sterilization base body 320, and each of the main germicidal lamps 310 can emit sterilization light toward the inner center direction and the outer direction.

Since the main germicidal lamp 310 can emit the sterilization light not only in the outer direction but also in the inner center direction, it may be configured to perform the air sterilization function of the circulation purification module 200 disposed in the inner center direction of the main germicidal lamp 310.

The main germicidal lamp 310 may be made of a material that allows light to transmit through all the outer circumferential surfaces of the lamp, so as to emit the sterilization light at 360° in all directions.

For example, the air purification case 210 of the circulation purification module 200 may be made of a transparent material, and the UV sterilization module 230 that emits sterilization light into the interior space of the air purification case 210 is not separately provided, so as to emit the sterilization light into the interior space of the air purification case 210 by the main germicidal lamp 310 of the sterilization and disinfection module 300. In this case, the sterilization light in the outer direction of the main germicidal lamp 310 performs the sterilization and disinfection function on the surface of the object, and the sterilization light in the inner direction performs the sterilization and disinfection function on the microbial particles passing through the air purification case 210.

Even if it is configured that the sterilization light of the main germicidal lamp 310 is emitted in both the outer and inner directions as described above, of course the above-described UV sterilization module 230 may be separately mounted inside the air purification case 210.

The indoor air sterilization and purification apparatus 30 according to the embodiment of the present invention may simultaneously perform the space sterilization mode and the surface sterilization mode as described above. In a process of the surface sterilization mode on the surface of the object 20, it is configured that the surface of the object 20 is irradiated with the sterilization light emitted from the main germicidal lamp 310 at a position close thereto.

For example, in the process of the surface sterilization mode on the lower surface of the object 20 to be disinfected existing in the indoor space, the controller 500 may control operations of the inventive apparatus so that, the lower surface of the object 20 is irradiated with the sterilization light, by moving the sterilization base body 320 upward so as to be spaced apart from the lower surface of the object 20 by a reference distance d, and in this state, the lower surface of the object 20 is irradiated with the sterilization light by horizontally pivoting and moving the main germicidal lamp 310 about the horizontal rotation shaft 340 by a pivot driving unit 360. In this case, the irradiation may be variously adjusted depending on a spatial arrangement state of the object 20 to be disinfected, such as all the plurality of main germicidal lamps 310 may be horizontally pivoted, and only some of the lamps may be selectively horizontally pivoted.

In addition, in this state, the sterilization base body 320 may be operated by a control of the controller 500 to be rotated about the vertical rotation shaft 350 by the main driving unit 330.

Depending on the operating state of the sterilization and disinfection module 300, since the lower surface of the object 20 may be irradiated with the sterilization light in close proximity by a reference distance d, the sterilization and disinfection function on the lower surface of the object 20 may be improved. In addition, since the main germicidal lamp 310 is rotated about the vertical rotation shaft 350, a relatively large region may be simultaneously irradiated with the sterilization light, and thereby decreasing the sterilization time.

Meanwhile, in the process of the surface sterilization mode on the upper surface of the object 20 to be disinfected existing in the indoor space, the controller 500 may control operations of the inventive apparatus so that the upper surface of the object 20 is proximately irradiated with the sterilization light emitted from the main germicidal lamp 310 by moving the sterilization base body 320 upward so as to be spaced apart from the upper surface of the object 20 by a temporary set distance d1, and in this state, horizontally pivoting and moving the main germicidal lamp 310, and then, moving the sterilization base body 320 downward so as to be spaced apart from the upper surface of the object 20 by a reference distance d shorter than the temporary set distance d1.

At this time, all the plurality of main germicidal lamps 310 may be horizontally pivoted and moved, but only some of the lamps may be moved close to the upper surface of the object 20 with being horizontally pivoted and moved, and in this state, the inventive apparatus may be controlled so that the entire region of the upper surface of the object 20 is irradiated with the sterilization light by moving the driving unit 100.

By such various control methods, even if the shape and arrangement state of the object 20 in the indoor space is complicated, the upper and lower surfaces of the object 20 may be proximately irradiated with the sterilization light without any special obstacle on the moving route, thereby improving the sterilization function.

According to the UV radiation apparatus of the present invention, it was confirmed that a generation of ozone in the atmosphere outside the apparatus might be suppressed. This is presumed to be that the generation of ozone by the light emitted from the irradiation window was suppressed by sufficiently absorbing light having a wavelength of 190 nm or less in the oxygen-containing layer. In addition, it was confirmed that the ozone filter of the exhaust fan may also reduce the ozone concentration to some extent. This is presumed to be because the ozone generated in the oxygen-containing layer formation space opened to the lamp housing unit was removed by the ozone filter in the process of being exhausted by the exhaust fan. It was found that, when the distance between the excimer lamp and the irradiation window was narrow, the ozone concentration was high in the vicinity of the irradiation window. This is presumed to be because light having a wavelength of 190 nm or less, which was not sufficiently absorbed due to insufficient thickness of the oxygen-containing layer, was emitted outside the apparatus.

According to the UV sterilization apparatus of the present invention, it was confirmed that B wavelength light might be removed, while a sufficiently large luminous intensity for A wavelength light could be obtained. This is presumed to be because, when the light guide unit is made of quartz glass, effective UV rays are reflected, but when a black paint is applied, the effective UV rays are partially absorbed.

In addition, it has been found that, when the incidence angle of the band pass filter is large, a large luminous intensity may be obtained with respect to effective UV rays, but it is difficult to remove harmful UV rays. This is presumed to be because the B wavelength light contained in the high-angle component light associated with the band pass filter is transmitted through the band pass filter by the reflective layer made of aluminum in the light guide unit, and is reflected as it is by the inner surface of the light guide unit, and then is emitted together with the A wavelength light from the irradiation window.

When conducting an experiment in which cells are irradiated with UV rays using the sterilization apparatus of the present invention, 222 nm has a protein absorption coefficient more than 10 times higher than 254 nm, and at 220 nm, when the skin thickness is 5 um or more, it exhibits a protein transmittance of 0.01% or less at UV-C transmittance according to the skin thickness of 27.4%, but at 255 nm, it appears that the protein transmittance is decreased to less than 0.1% when the skin thickness is 60 um or more.

In an experiment on bactericidal power, it can be seen that, as for bacteria, when comparing the bactericidal power against live bacteria and spores in strains such as Methicillin-Resistant Staphylococcus aureus, Pseudomonas aeruginosa, Escherichia Coli O157, Salmonella Typhimurium, Campylobacter jejuni, Bacillus cereus, Bacillus subtilis, Clostrium difficile, it exhibits sterilization ability at 222 nm which is equivalent to or more improved sterilization ability than 254 nm.

Further, it can be seen that, as for fungi and yeast, when comparing the bactericidal power against cells and spores of Candida albicans, Penicillium expansum, and Aspergillus niger, it exhibits sterilization ability at 222 nm which is equivalent to or better improved sterilization ability than 254 nm.

Furthermore, it can be seen that, as for the virus, it exhibits sterilization ability at 222 nm which is equivalent to or better improved sterilization ability than 254 nm in relation to bacteriophage MS2 and Feline Calicivirus. Thus, it exhibits high bactericidal power 222 nm in terms of all the bacteria, fungi and viruses.

A schematic view and an embodiment of the dielectric barrier discharge of the present invention are photographed, and shown in FIGS. 4A and 4B. It can be seen that, when discharging the dielectric barrier discharge, it evenly emits light in a thread-shaped light emission form different from the RF discharge that uniformly emits light.

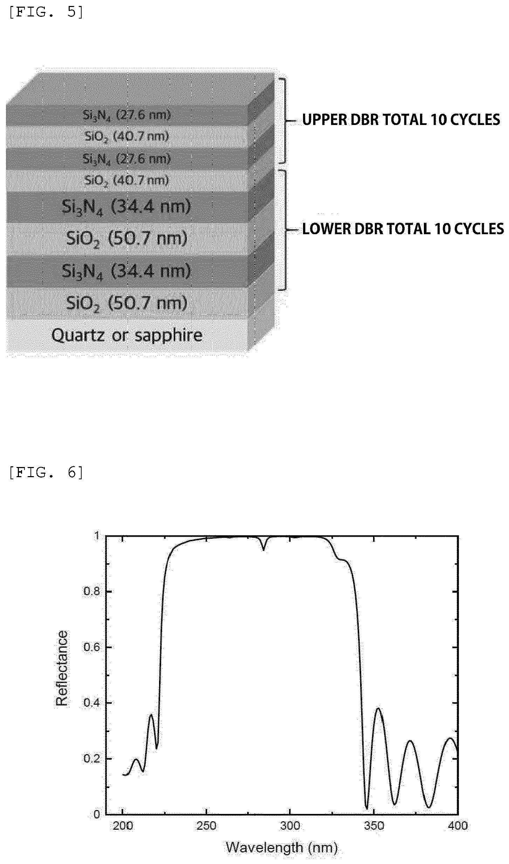

A schematic view of the DBR filter of the present invention is shown in FIG. 5.

When the upper DBR filter is formed in a 20-layerd structure having a Si3N4 (27.6 nm) and SiO2 (40.7 nm) as a repeatable laminated structure, and the lower DBR filter is formed in a 20-layerd structure having a Si3N4 (34.4 nm) and SiO2 (50.7 nm) as a repeatable laminated structure, the obtained light reflectance is shown in FIG. 6. By reflecting almost 100% of light having a wavelength of about 223 nm to 345 nm, only wavelength of 222 nm or less can be selectively irradiated.

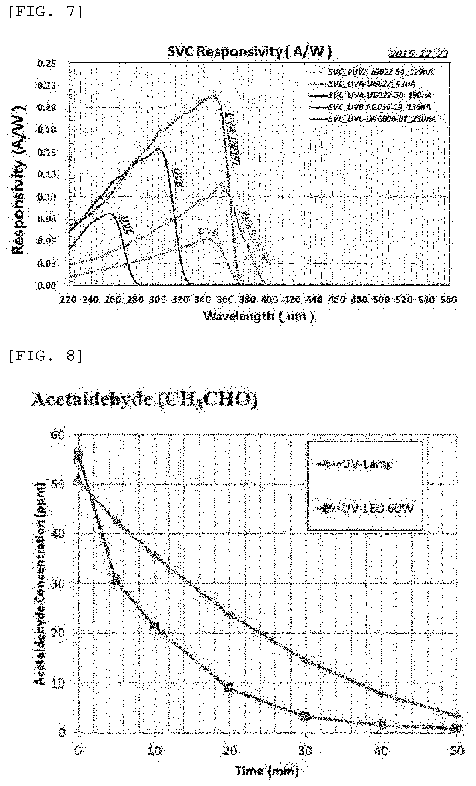

Results of the sensitivity experiment of UV photo diode (purchased from Seoul Viosys) of the present invention are shown in FIG. 7. Conventionally, the maximum sensitivity was 0.09 A/W in UVC, 0.15 A/W in UVB, and 0.05 A/W in UVA, respectively. The UV photo diode of the present invention has a remarkably improved sensitivity, thereby exhibiting 0.215 A/W in UVA, and the maximum sensitivity of 0.115 A/W in PUVA, which were conventionally difficult to measure.

Results of the acetaldehyde removal experiment of the present invention are shown in FIG. 8. It can be seen that the ozone removal device of the present invention improves the aldehyde removal ability by more than twice per hour than the conventional UV-lamp.

Although the sterilization apparatus according to the embodiments of the present invention has been described above, but the sterilization apparatus according to the embodiments of the present invention is not limited to the above-described embodiments, and various modifications may be added.

[Description of Reference Numerals]

| | |
|---|---|
| 100: | Driving unit |
| 200: | Circulation purification module |
| 210: | Air purification case |
| 211: | Air inlet |
| 212: | Air outlet |
| 220: | Air filter |
| 230: | Air germicidal lamp |
| 240: | Blowing fan |
| 300: | Sterilization and disinfection module |
| 310: | Main germicidal lamp |
| 320: | Sterilization base body |
| 330: | Main driving unit |
| 340: | Horizontal rotation shaft |
| 350: | Vertical rotation shaft |
| 360: | Rotation driving unit |
| 400: | Distance detection sensor |
| 500: | Controller |

**Claims**

1. An ultraviolet (UV) sterilization module comprising:

    a UV light source unit configured to emit light including UV rays having a wavelength of 200 to 230 nm;
    a housing unit configured to house the UV light source; and
    a light guide unit which is configured to guide the light from the UV light source, and is blocked by an irradiation window allowing transmittance of UV rays,
    wherein the UV light source unit includes a DBR filter configured to selectively emit only peaks including a wavelength of 222 nm.

2. The UV sterilization module according to claim 1, wherein the UV light source is one or more selected from the group consisting of an excimer lamp, an arc discharge lamp, or a UV-C LED.

3. The UV sterilization module according to claim 2, wherein the excimer lamp comprises one or more excimers selected from the group consisting of ArF, KrBr, KrCl, KrF, and Xel.

4. The UV sterilization module according to claim 1, wherein the UV sterilization module is configured to directly irradiate skin with a radiation light thereof.

5. An ultraviolet (UV) sterilization apparatus comprising the UV sterilization module according to any one of claims 1 to 4.

6. An ultraviolet (UV) sterilization method comprising:

    performing, by the UV sterilization apparatus according to claim 5, one or more of sterilization, deodorization, and removal of organic contaminants by directly irradiating skin of human or animal with UV rays emitted from the UV sterilization apparatus.

7. The UV sterilization module according to claim 2, wherein a light emission method of the excimer lamp may be a light emission method of dielectric barrier discharge.

8. The UV sterilization module according to claim 1, the DBR filter comprises an upper DBR and a lower DBR in a laminated structure, and
    a material of each layer of the upper DBR and the lower DBR is selected from the group consisting of Si3N4, SiO2, or ZnO.

9. The UV sterilization module according to claim 7, wherein each layer of the upper DBR and the lower DBR has a thickness of 5 nm to 500 nm.

10. The UV sterilization module according to claim 7, wherein each of the upper DBR and the lower DBR has a laminated structure of 5 to 100 layers.

11. The UV sterilization module according to claim 2, wherein one or more moth eye lenses are installed on one or more of LED modules of the UV-C LED light source.

12. The UV sterilization apparatus according to claim 5, wherein the UV sterilization apparatus further comprises a safety device,
    wherein the safety device includes a detector configured to measure one or more wavelength lights selected from the group consisting of UVC, UVB, UVA, and PUVA.

13. The UV sterilization apparatus according to claim 5, wherein the UV sterilization apparatus further comprises an ozone removal device, and
    the ozone removal device comprises a UV radiation unit and a photocatalytic filter.

[FIG. 1]

[FIG. 2]

[FIG. 3]

[FIG. 4a]

RF discharge

Dielectric material (glass, etc.)     External electrode

Glow discharge

Dielectric barrier discharge

Dielectric material (glass, etc.)     External electrode

Microplasma discharge

[FIG. 4b]

[FIG. 5]

[FIG. 6]

[FIG. 7]

**SVC Responsivity ( A/W )**   _2015. 12. 23_

[FIG. 8]

**Acetaldehyde (CH$_3$CHO)**

Europäisches
Patentamt

European
Patent Office

Office européen
des brevets

# EUROPEAN SEARCH REPORT

Application Number

EP 20 20 7974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2016/196904 A1 (UNIV COLUMBIA [US]) 8 December 2016 (2016-12-08) * paragraphs [0024], [0039] * | 1-12 | INV. A61L2/00 A61L2/10 |
| Y | WO 2016/044759 A1 (XENEX DISINFECTION SERVICES LLC [US]) 24 March 2016 (2016-03-24) * paragraphs [0003], [0004], [0022], [0028], [0037], [0044], [0045], [0059], [0070], [0072], [0077]; claims; figures * | 1-3,5, 7-13 | |
| Y | JP 2003 163393 A (NIKON CORP) 6 June 2003 (2003-06-06) * paragraphs [0028], [0042] - [0047], [0067], [0073]; figures 1, 6 * | 1-13 | |
| Y | KR 2003 0093140 A (CHO YONG IK [KR]; KIM BYUNG HOON [KR] ET AL.) 6 December 2003 (2003-12-06) * page 3(4), paragraph 11; figures 2-6 * | 11 | |
| A | | 1-10,12, 13 | TECHNICAL FIELDS SEARCHED (IPC) A61L |
| A | Ulrich Kogelschatz: "Dielectric-Barrier Discharges: Their History, Discharge Physics, and Industrial Applications", Plasma Chemistry and Plasma Processing, 1 March 2003 (2003-03-01), pages 1-46, XP055430248, New York DOI: 10.1023/A:1022470901385 Retrieved from the Internet: URL:https://www3.nd.edu/~sst/teaching/AME6 0637/reading/2003_PCPP_Kogelschatz_dbd_rev iew.pdf [retrieved on 2021-04-30] * paragraphs [0007], [07.1], [07.2] * | 7 | |

The present search report has been drawn up for all claims

| Place of search | Date of completion of the search | Examiner |
|---|---|---|
| Munich | 14 May 2021 | Serra, Renato |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 7974

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2021

| Patent document cited in search report | | Publication date | Patent family member(s) | | Publication date |
|---|---|---|---|---|---|
| WO 2016196904 | A1 | 08-12-2016 | CN | 107613895 A | 19-01-2018 |
| | | | CN | 111888514 A | 06-11-2020 |
| | | | EP | 3302328 A1 | 11-04-2018 |
| | | | JP | 6847053 B2 | 24-03-2021 |
| | | | JP | 2018517488 A | 05-07-2018 |
| | | | US | 2020215215 A1 | 09-07-2020 |
| | | | WO | 2016196904 A1 | 08-12-2016 |
| WO 2016044759 | A1 | 24-03-2016 | AU | 2015317384 A1 | 04-05-2017 |
| | | | AU | 2018202610 A1 | 10-05-2018 |
| | | | AU | 2020207856 A1 | 13-08-2020 |
| | | | CA | 2961224 A1 | 24-03-2016 |
| | | | CN | 106998764 A | 01-08-2017 |
| | | | EP | 3193634 A1 | 26-07-2017 |
| | | | EP | 3335573 A1 | 20-06-2018 |
| | | | EP | 3718413 A1 | 07-10-2020 |
| | | | ES | 2767401 T3 | 17-06-2020 |
| | | | ES | 2811360 T3 | 11-03-2021 |
| | | | GB | 2545375 A | 14-06-2017 |
| | | | GB | 2558367 A | 11-07-2018 |
| | | | HK | 1258131 A1 | 08-11-2019 |
| | | | JP | 6313523 B2 | 18-04-2018 |
| | | | JP | 2017530777 A | 19-10-2017 |
| | | | JP | 2018102976 A | 05-07-2018 |
| | | | KR | 20170046799 A | 02-05-2017 |
| | | | KR | 20180040733 A | 20-04-2018 |
| | | | KR | 20190088570 A | 26-07-2019 |
| | | | KR | 20200067906 A | 12-06-2020 |
| | | | KR | 20210016092 A | 10-02-2021 |
| | | | PL | 3335573 T3 | 16-11-2020 |
| | | | RU | 2663459 C1 | 06-08-2018 |
| | | | RU | 2018127141 A | 14-03-2019 |
| | | | SG | 10201902236T A | 29-04-2019 |
| | | | SG | 11201808015V A | 30-10-2018 |
| | | | US | 2017173195 A1 | 22-06-2017 |
| | | | US | 2018272017 A1 | 27-09-2018 |
| | | | US | 2019209722 A1 | 11-07-2019 |
| | | | WO | 2016044759 A1 | 24-03-2016 |
| JP 2003163393 | A | 06-06-2003 | NONE | | |
| KR 20030093140 | A | 06-12-2003 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200137298

**Non-patent literature cited in the description**

- **DAVID WELCH ; MANUELA BUONANNO ; VELJKO GRILJ ; IGOR SHURYAK ; CONNOR CRICKMORE ; ALAN W. BIGELOW ; GERHARD RANDERS-PEHRSON ; GARY W. JOHNSON ; DAVID J. BRENNER.** Far-UVC light: A new tool to control the spread of airborne-mediated microbial diseases. *Scientific Reports,* 2018, vol. 8